# EUROPÄISCHE PATENTSCHRIFT

(11) **EP 3 356 787 B1**
(45) Veröffentlichungstag und Bekanntmachung des Hinweises auf die Patenterteilung: **04.03.2020**
(21) Anmeldenummer: 16778182.2
(22) Anmeldetag: 22.09.2016
(51) Int. Cl.: G01N 11/08, G01N 33/28

(54) **VERFAHREN UND VORRICHTUNG ZUR BESTIMMUNG DER NIEDERTEMPERATUREIGENSCHAFTEN**
METHOD AND DEVICE FOR DETERMINING LOW TEMPERATURE PROPERTIES
PROCÉDÉ ET DISPOSITIF POUR DÉTERMINER LES PROPRIÉTÉS À BASSE TEMPÉRATURE

(30) Priorität: 29.09.2015 AT 6362015
(43) Veröffentlichungstag der Anmeldung: 08.08.2018
(73) Patentinhaber: Grabner Instruments Messtechnik GmbH, 1220 Wien (AT)
(72) Erfinder: GLANZNIG, Gerd, 1020 Wien (AT); LUTZ, Josef, 2471 Rohrau (AT)
(74) Vertreter: Keschmann, Marc
(86) Internationale Anmeldenummer: PCT/AT2016/000084
(87) Internationale Veröffentlichungsnummer: WO 2017/054019

(56) Entgegenhaltungen:
- WO-A1-2015/041672
- JP-A- H10 274 616
- US-A- 3 122 911
- US-A- 4 292 837
- US-A1- 2011 083 994

## Beschreibung

Die Erfindung betrifft ein Verfahren zur Bestimmung der Niedertemperatureigenschaften eines paraffinhaltigen Brennstoffs gemäß Anspruch 1, bei dem der Brennstoff aus einer Vorratskammer durch eine mit einem Sieb versehene Messzelle geleitet wird, die Messzelle mittels einer Kühleinrichtung gekühlt wird, die Temperatur des in der Messzelle befindlichen Brennstoffs gemessen und ein den am Sieb auftretenden Strömungswiderstand repräsentierender Flüssigkeitsdruck gemessen wird und die bei einem definierten Sollwert des Flüssigkeitsdrucks auftretende Temperatur ermittelt und als Ergebnis des Verfahrens ausgegeben wird.

Gemäß Anspruch 14 betrifft die Erfindung weiters eine Vorrichtung zur Durchführung eines solchen Verfahrens.

Ein Verfahren und eine Vorrichtung der eingangs genannten Art sind in der DD 120714 beschrieben. Das Verfahren dient insbesondere der Bestimmung der Filtrierbarkeitsgrenze eines paraffinhaltigen Brennstoffs.

Bei niedrigen Temperaturen haben Mineralölmitteldestillate die Eigenschaft, ein schlechtes Fließverhalten zu zeigen und Festparaffin auszuscheiden. Wenn beispielsweise ein Dieselkraftstoff mit einem Trübungspunkt von 0°C über eine längere Zeitdauer unterhalb 0°C gehalten wird, z.B. während einer Kälteperiode im Winter, kristallisiert das Paraffin in tafelartigen Kristallen aus, die den Kraftstoff gelieren lassen und seinen Durchgang durch enge Leitungen und Filter verhindern.

Die Temperatur der Filtrierbarkeitsgrenze oberhalb derer flüssige Mineralölprodukte noch störungsfrei eingesetzt werden können, ist daher ein geeignetes Gütekriterium für die Verwendbarkeit dieser Stoffe in kalten Klimazonen oder im Winterbetrieb. Es ist bekannt, die Bestimmung der Temperatur der Filtrierbarkeitsgrenze derart durchzuführen, dass man eine abgekühlte Probe des Brennstoffs in regelmäßigen Temperaturintervallen durch ein Metallsieb genormter Maschenweite in einen Auffangbehälter drückt (ASTM D6371). Nach Durchtritt der festgelegten Menge läuft die Probe unter dem Einfluss der Schwerkraft in die Kühlzelle zurück. Die Temperatur der Filtrierbarkeitsgrenze gilt als erreicht, wenn die Durchflusszeit im Filtriertakt einen bestimmten Wert (beispielsweise 60 sec) überschreitet.

In einer anderen besonders für europäische und additivhaltige Dieselkraftstoffe empfohlenen Ausführungsform des Filtrierbarkeitstestes (Cold-Filter- Plugging-Point-Test / CFPP-Test) ermittelt man in einer Folge regelmäßig wiederholter Saug- und Belüftungstakte die Grenztemperatur, bei der eine stetig gekühlte Kraftstoffprobe ein direkt in der Kühlzelle angebrachtes Prüfsieb gerade nicht mehr passiert.

Während Verfahren mit Saug- und Belüftungstakte diskontinuierlich arbeiten, wurde in der DD 120714 ein kontinuierliches Verfahren vorgeschlagen, bei dem die mit einem Metallsieb festgelegter Maschenweite ausgestattete Kühl- bzw. Messzelle als Durchflusszelle ausgebildet ist und kontinuierlich in nur einer Richtung von dem zu überprüfenden Brennstoff durchflossen wird. Der Strömungswiderstand, den das Metallsieb für den Produktstrom besitzt, wird durch den Druck am Eingang der Messzelle ermittelt. Die Messzelle ist mit an deren Seitenflächen angebrachten Peltier-Kühlbatterien ausgestattet, deren Kühlleistung in Abhängigkeit des Druckmesswerts geregelt wird. Die Regelung erfolgt so, dass sich durch Ausscheidung einer bestimmten Menge von Paraffinkristallen am Metallsieb der vorgewählte Druck einstellt, der mit der Temperatur der Filtrierbarkeitsgrenze des untersuchten Brennstoffs korrespondiert. Die dabei von einem Messfühler ermittelte Probentemperatur ist die gewünschte Filtrierbarkeitsgrenze.

Nachteilig bei dem in der DD 120714 beschriebenen Verfahren ist, dass exakte Messergebnisse nur dann erreicht werden, wenn bei der Inbetriebnahme der Prüfeinrichtung ein vorgegebener Mengenstrom der Probe eingestellt und dieser während des gesamten Verfahrens gleich gehalten wird. Der Grund hierfür liegt darin, dass Änderungen des Mengenstroms die Ergebnisse der Druckmessung verfälschen. Ein weiterer Nachteil des Verfahrens gemäß der DD 120714 liegt darin, dass ein konstanter Fluss der Probeflüssigkeit benötigt wird, sodass ein großes Volumen der Probe verbraucht wird.

Das Dokument US 4292837 A offenbart eine Testvorrichtung zur Bestimmung der Fließeigenschaften von Kerosin.

Das Dokument WO 2015/041672 A1 offenbart ein System zum Ermitteln des Trübungspunktes einer Flüssigkeit, wobei der Druck mehrmals bei unterschiedlichen Temperaturen gemessen und daraus eine Kennlinie erstellt wird.

Die Erfindung zielt daher darauf ab, ein Verfahren und eine Vorrichtung der eingangs genannten Art dahingehend zu verbessern, dass der definierte Mengenstroms in einfacher Weise eingestellt und gleichgehalten und das verbrauchte Probenvolumen verringert werden kann.

Zur Lösung dieser Aufgabe sieht die Erfindung bei einem Verfahren der eingangs genannten Art vor, dass für jede Druckmessung eine definierte Probemenge des Brennstoffs stoßartig aus der Vorratskammer gefördert wird, um einen Druckpuls zu erhalten. Dadurch, dass ein Druckpuls zur Messung des Flüssigkeitsdrucks herangezogen wird, ist ein ständiger Mengenstrom des Brennstoffs nicht erforderlich, sodass das Volumen des für das Verfahren erforderlichen Brennstoffs reduziert werden kann. Der gewünschte Mengenstrom ergibt sich hierbei aus einer Kontrolle der jeweils ausgestoßenen Brennstoffmenge und der Zeit, über welche der Ausstoß erfolgt. Bei einem kurzfristigen Ausstoß einer geringen Brennstoffmenge lässt sich die Einstellung und Kontrolle des Mengenstroms technisch wesentlich einfacher realisieren als bei einer konstanten Brennstoffförderung.

Bevorzugt wird hierbei so vorgegangen, dass die Messung des Flüssigkeitsdrucks während des Kühlens der Messzelle bei einer Reihe unterschiedlicher Temperaturen des Brennstoffs wiederholt wird, um eine Messwertreihe zu erhalten, wobei für jede Messung eine definierte, gleiche Probemenge des Brennstoffs stoßartig aus der Vorratskammer gefördert wird, um einen Druckpuls zu erhalten. Dadurch, dass die Druckmessung während des Abkühlens des Brennstoffs nicht kontinuierlich erfolgt, sondern eine Messwertreihe aus in bestimmten Abständen vorgenommenen Druckmesswerten ermittelt wird, ist ein ständiger Mengenstrom des Brennstoffs nicht erforderlich, sodass das Volumen des für das Verfahren erforderlichen Brennstoffs reduziert werden kann. Die Einhaltung des geforderten Mengenstroms erfolgt hierbei in einfacher Weise dadurch, dass für jede Messung eine definierte, gleiche Probemenge des Brennstoffs stoßartig aus der Vorratskammer gefördert wird, um einen Druckpuls zu erhalten. In bevorzugter Weise wird der Ausstoß einer definierten Brennstoffmenge jeweils dadurch erreicht, dass ein Förderkolben einer Fördereinrichtung mittels einer Antriebseinrichtung, insbesondere eines Schrittmotors um einen definierten Weg verschoben wird. Alternativ kann die definierte Brennstoffmenge auch mittels einer Mikropumpe gefördert werden.

Der kurzfristige Ausstoß der definierten Probemenge des Brennstoffs verursacht stromabwärts der Fördervorrichtung bzw. stromaufwärts des in der Messzelle angeordneten Siebs auf Grund der Kompressibilität des Brennstoffs einen Druckpuls. Der Druckpuls wird von der Druckmesseinrichtung erfasst, wobei bevorzugt so vorgegangen wird, dass das Maximum des bei einem Druckpuls auftretenden Flüssigkeitsdrucks jeweils als Messwert des Flüssigkeitsdrucks herangezogen wird.

Der am Sieb auftretende Strömungswiderstand kann grundsätzlich durch verschiedene Druckmessungen repräsentiert werden. Zum Beispiel kann ein Differenzdruck von vor und hinter dem Sieb gemessenen Druckwerten als der den Strömungswiderstand repräsentierende Flüssigkeitsdruck herangezogen werden. In einfacher Weise wird bevorzugt aber so vorgegangen, dass der stromaufwärts der Messzelle herrschende Flüssigkeitsdruck als der den am Sieb auftretenden Strömungswiderstand repräsentierende Flüssigkeitsdruck herangezogen wird.

Der durch das erfindungsgemäße Verfahren zu ermittelnde, die Niedertemperatureigenschaft des Brennstoffs kennzeichnende Parameter, wie insbesondere die Filtrierbarkeitsgrenze bzw. der CFPP, wird grundsätzlich so ermittelt, dass zunächst eine Kalibrierung stattfindet, bei welcher anhand eines Brennstoffs mit bekanntem Temperaturwert des Parameters, insbesondere der Filtrierbarkeitsgrenze, derjenige Flüssigkeitsdruck ermittelt wird, der bei dem bekannten Temperaturwert des Parameters dieses Brennstoffs gemessen wird. Dieser Druck-Messwert wird dann für die betreffende Prüfeinrichtung in Bezug auf den betreffenden Parameter als definierter Sollwert des Flüssigkeitsdrucks festgelegt. Die Kalibrierung kann bei nichtlinearen Zusammenhängen zwischen der Filtrierbarkeitsgrenze und dem Flüssigkeitsdruck durch eine Korrelation, die den nichtlinearen Zusammenhang berücksichtigt, ersetzt werden. Sobald nun bei Durchführung des Testverfahrens mit einem Brennstoff mit unbekanntem Parameter der Niedertemperatureigenschaft der definierte Sollwert des Flüssigkeitsdrucks erreicht wird, wird die aktuelle Temperatur des in der Messzelle befindlichen Brennstoffs ermittelt und als gesuchter Temperaturwert des Parameters, wie z.B. der Filtrierbarkeitsgrenze ausgegeben.

Mit Rücksicht auf den Umstand, dass im Rahmen des erfindungsgemäßen Verfahrens kein kontinuierliches Druckmesssignal, sondern eine Messwertreihe aus diskreten Druckmesswerten erhalten wird, kann der gewünschte Parameter, wie z.B. die Temperatur der Filtrierbarkeitsgrenze dann als bestimmt gelten, wenn der aktuelle Flüssigkeitsdruckmesswert den Sollwert erstmals überschreitet.

Eine Erhöhung der Genauigkeit wird gemäß einer bevorzugten Verfahrensweise aber erreicht, wenn aus der Messwertreihe eine Kennlinie des Flüssigkeitsdrucks in Abhängigkeit von der Temperatur erstellt wird und die in der Kennlinie dem definierten Sollwert des Flüssigkeitsdrucks zugeordnete Temperatur ermittelt und als Ergebnis des Verfahrens ausgegeben wird.

Das erfindungsgemäße Verfahren eignet sich nicht nur zur Bestimmung der Filtrierbarkeitsgrenze bzw. des Cold Filter Plugging Point (CFPP) des Brennstoffs, sondern auch zur Bestimmung des Pour Point des Brennstoffs. Der Pour Point des Brennstoffs ist diejenige Temperatur, bei welcher dieser bei Abkühlung gerade noch fließt. Für die Definition der Fließeigenschaft geben die Normen (DIN, ASTM) verschiedene Prüfverfahren an.

Mit dem erfindungsgemäßen Verfahren lassen sich sowohl die Filtrierbarkeitsgrenze als auch der Pour Point in einem einzigen Verfahrensdurchlauf ermitteln. Es ist zu diesem Zweck bevorzugt vorgesehen, dass ein erster Sollwert des Flüssigkeitsdrucks vorgegeben wird, der für die Filtrierbarkeitsgrenze bestimmend ist und dass ein zweiter Sollwert des Flüssigkeitsdrucks vorgegeben wird, der für den Pour Point bestimmend ist. Sobald in einem Verfahrensdurchlauf die Messwertreihe erhalten wurde, kann im Rahmen der Auswertung der Messwertreihe oder der aus der Messwertreihe erzeugten Kennlinie anhand des ersten Sollwerts die Filtrierbarkeitsgrenze und anhand des zweiten Sollwerts der Pour Point bestimmt werden.

Das Verfahren kann so durchgeführt werden, dass die Temperatur des in der Messzelle befindlichen Brennstoffs stufenweise reduziert wird, insbesondere in Stufen von 1°C, und dass nach jeder Abkühlstufe eine Messung des Flüssigkeitsdrucks erfolgt.

Alternativ kann die Temperatur des in der Messzelle befindlichen Brennstoffs kontinuierlich reduziert werden und beim Durchlaufen von definierten Temperaturstufen jeweils eine Messung des Flüssigkeitsdrucks erfolgen. Die kontinuierliche Reduzierung der Temperatur ist hierbei bevorzugt, weil das Verfahren schneller beendet ist.

Auf Grund der Ausbildung der Messzelle als Durchflusszelle kann zusätzlich zur Filtrierbarkeitsgrenze und/oder zum Pour Point auch der Trübungspunkt (Cloud Point) und/oder der Freeze Point des Brennstoffs ermittelt werden. Dies erfolgt bevorzugt in der Messzelle mittels eines optischen Messverfahrens. Das optische Messverfahren umfasst bevorzugt eine Durchlichtmessung.

Der Trübungspunkt ist ebenfalls eine Kälteeigenschaft von Dieselkraftstoff und Heizöl und bezeichnet diejenige Temperatur (°C), bei der ein blankes, flüssiges Produkt unter definierten Prüfbedingungen durch die Bildung von Paraffinkristallen trüb oder wolkig wird.

Wenn sowohl die Filtrierbarkeitsgrenze und/oder der Pour Point als auch der Trübungspunkt in einem einzigen Verfahrensdurchlauf ermittelt werden sollen, wird bevorzugt so vorgegangen, dass der jeweils in der Messzelle befindliche Brennstoff gekühlt wird und während eines ersten Kühlschrittes der Trübungspunkt und während eines zweiten Kühlschrittes die Filtrierbarkeitsgrenze und/oder der Pour Point ermittelt wird.

Zusätzlich kann in einfacher Weise auch der Freeze Point des Brennstoffs ermittelt werden. Der Freeze Point definiert gemeinsam mit dem Cloud Point das Schmelzintervall. Bevorzugt wird hierbei so vorgegangen, dass der jeweils in der Messzelle befindliche Brennstoff nach der Kühlung wieder erwärmt wird und während der Erwärmung der Freeze Point ermittelt wird.

Gemäß einem weiteren Aspekt der Erfindung wird die der Erfindung zugrunde liegende Aufgabe durch eine Vorrichtung zur Durchführung des erfindungsgemäßen Verfahrens gelöst, umfassend eine Vorratskammer für den zu prüfenden paraffinhaltigen Brennstoff, eine mit der Vorratskammer in Leitungsverbindung stehende, als Durchflusszelle ausgebildete und mit einem Sieb versehene Messzelle, eine Fördervorrichtung zum Fördern von Brennstoff aus der Vorratskammer durch die Messzelle, eine Kühlvorrichtung zum Kühlen der Messzelle, einen Temperatursensor zum Messen der Temperatur des in der Messzelle befindlichen paraffinhaltigen Brennstoffs, einen Drucksensor zum Messen eines den am Sieb auftretenden Strömungswiderstand repräsentierenden Flüssigkeitsdrucks und eine Steuereinheit mit einer Auswerteschaltung, der die Messwerte des Temperatursensors und des Drucksensors zugeführt sind, wobei die Fördervorrichtung zum stoßartigen Fördern einer definierten Probemenge des paraffinhaltigen Brennstoffs aus der Vorratskammer ausgebildet ist, um einen Druckpuls zu erhalten, und wobei die Auswerteschaltung eingerichtet ist, die bei einem definierten Sollwert des Flüssigkeitsdrucks auftretende Temperatur zu ermitteln und als Ergebnis auszugeben.

Die Steuereinheit kann beispielsweise als Mikrokontroller ausgebildet sein.

Der Druckpuls wird bevorzugt dadurch erzeugt, dass die Fördervorrichtung einen die Vorratskammer begrenzenden Kolben umfasst, der mittels einer Antriebseinrichtung betätigbar ist. Insbesondere ist vorgesehen, dass die Antriebseinrichtung einen Schrittmotor umfasst. Alternativ kann die Fördervorrichtung eine Mikropumpe oder andere Aktuatoren wie z.B. Piezo-Aktuatoren umfassen.

Bevorzugt ist vorgesehen, dass der Drucksensor zur Messung des stromaufwärts der Messzelle herrschenden Flüssigkeitsdrucks angeordnet ist. In konstruktiv besonders vorteilhafter Weise ist der Drucksensor hierbei in den Kolben der Fördervorrichtung integriert.

Bevorzugt ist vorgesehen, dass die Steuereinheit mit der Kühlvorrichtung und der Fördervorrichtung zusammenwirkt, um diese in Abhängigkeit von den Messwerten des Temperatursensors und des Drucksensors anzusteuern, und dass die Steuereinheit eingerichtet ist, um die Messung des Flüssigkeitsdrucks während des Kühlens der Messzelle bei einer Reihe unterschiedlicher Temperaturen des Brennstoffs zu wiederholen, wobei eine Messwertreihe erhalten wird, und um für jede Messung eine definierte, gleiche Probemenge des Brennstoffs stoßartig aus der Vorratskammer zu fördern.

Um die Messwertepaare der Messwertreihe verarbeiten zu können, ist erfindungsgemäß vorgesehen, dass die Steuereinheit eine Auswerteschaltung umfasst, der die Messwerte des Drucksensors und des Temperatursensors zugeführt sind und die ggf. die Messwertreihe gespeichert hat, wobei die Auswerteschaltung die bei einem definierten Sollwert des Flüssigkeitsdrucks auftretende Temperatur ermittelt und als Ergebnis ausgibt.

Bevorzugt wird das Maximum des bei einem Druckpuls auftretenden Flüssigkeitsdrucks in der Auswerteschaltung jeweils als Messwert des Flüssigkeitsdrucks herangezogen.

In vorteilhafter Weise ist die Auswerteschaltung dabei eingerichtet, um aus der Messwertreihe eine Kennlinie des Flüssigkeitsdrucks in Abhängigkeit von der Temperatur zu erstellen und die in der Kennlinie dem definierten Sollwert des Flüssigkeitsdrucks zugeordnete Temperatur zu ermitteln und als Ergebnis auszugeben.

Die Steuereinheit kann mit der Kühlvorrichtung zur stufenweisen Reduzierung der Temperatur des in der Messzelle befindlichen Brennstoffs zusammenwirken, insbesondere in Stufen von 1°C, wobei nach jeder Abkühlstufe eine Messung des Flüssigkeitsdrucks erfolgt.

Alternativ ist bevorzugt vorgesehen, dass die Steuereinheit mit der Kühlvorrichtung zur kontinuierlichen Reduzierung der Temperatur des in der Messzelle befindlichen Brennstoffs zusammenwirkt und dass beim Durchlaufen von definierten Temperaturstufen jeweils eine Messung des Flüssigkeitsdrucks erfolgt.

Eine weitere bevorzugte Weiterbildung sieht vor, dass der Messzelle eine optische Messeinrichtung zur Messung des Trübungspunkts (Cloud Point) und/oder des Freeze Point des Brennstoffs zugeordnet ist. Insbesondere arbeitet die optische Messeinrichtung nach dem Durchlichtverfahren und umfasst vorzugsweise eine an der einen Seite der Messzelle angeordnete Lichtquelle und einen an der gegenüberliegenden Seite der Messzelle angeordneten Lichtsensor.

Zur Kühlung der Messzelle wird bevorzugt eine Kühleinrichtung mit einstellbarer Kühlleistung eingesetzt. Bevorzugt umfasst die Kühleinrichtung zumindest ein Peltier-Element. Die Messzelle kann hierbei entweder einseitig oder zweiseitig mit Peltier-Elementen versehen sein.

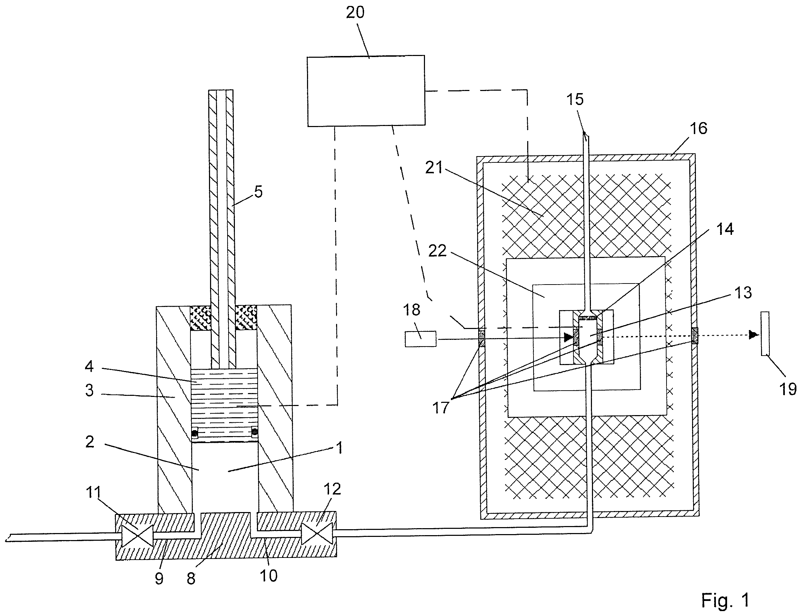

Die Erfindung wird nachfolgend anhand eines in der Zeichnung schematisch dargestellten Ausführungsbeispiels näher erläutert. In dieser zeigt Fig. 1 eine erfindungsgemäße Vorrichtung zur Bestimmung des Trübungspunktes und der Filtrierbarkeitsgrenze eines paraffinhaltigen Brennstoffs.

In Fig. 1 ist mit 1 eine Vorratskammer für den zu prüfenden Brennstoff bezeichnet, in der eine Probe 2 des Brennstoffs aufgenommen ist. Die Vorratskammer 1 ist in einem Hohlzylinder 3 ausgebildet, der auf der einen Seite durch einen axial verschieblichen Kolben 4 verschlossen ist. Der Kolben 4 weist eine Kolbenstange 5 auf, der mit einem nicht dargestellten Verschiebeantrieb für den Kolben 4 zusammenwirkt. Der Verschiebeantrieb kann beispielsweise von einem Schrittmotor gebildet sein. An der dem Kolben 4 gegenüberliegenden Seite ist die Vorratskammer 1 durch einen Ventilblock 8 verschlossen, in dem die Zuflussleitung 9 und die Abflussleitung 10 ausgebildet sind. Die Zuflussleitung 9 weist ein Ventil 11 und die Abflussleitung weist ein Ventil 12 auf.

Bei geöffnetem Ventil verbindet die Abflussleitung 10 die Vorratskammer 1 mit der Messzelle 13, die als Durchflusszelle ausgebildet und mit einem Sieb 14 versehen ist. Sobald der Kolben 4 eine definierte Probemenge des Brennstoffs stoßartig aus der Vorratskammer 1 fördert, gelangt die Probenmenge über die Leitung 10 in die Messzelle 13. Die Probe durchfließt die Messzelle 13 und verlässt diese somit an der der Zuleitung gegenüberliegenden Seite über den Abfluss 15. Ein den am Sieb 14 entstehenden Strömungswiderstand repräsentierender Druckwert wird durch einen Drucksensor ermittelt, der im vorliegenden Fall in den Kolben 4 integriert ist. Alternativ ist aber auch ein Drucksensor denkbar, der in der Zuleitung 10 oder an einer anderen, stromaufwärts der Messzelle 13 befindlichen Stelle angeordnet ist.

Zur Kühlung der Messzelle 13 ist eine Kühleinrichtung vorgesehen, wobei die Messzelle 13 samt der kalten Seite der Kühleinrichtung thermisch isoliert ist, wie mit 16 schematisch angedeutet.

Weiters ist in Fig. 1 ersichtlich, dass die Messzelle 13 an zwei gegenüberliegenden Seiten jeweils ein Glasfenster 17 aufweist. Die thermische Isolierung 16 weist ebenfalls entsprechende Glasfenster bzw. geeignete optische Durchführungen 17 auf, wobei alle optischen Elemente 17 miteinander fluchten. Auf der einen Seite der Messzelle 13 ist eine Lichtquelle 18, wie z.B. ein Laser, und auf der gegenüberliegenden Seite ist ein Photodetektor 19 angeordnet, und zwar so, dass das von der Lichtquelle 18 ausgesendete Licht die Glasfenster 17 sowie die Messzelle 13 durchqueren und vom Photodetektor 19 erfasst werden kann. Durch ein solches Durchlichtverfahren kann der Trübungspunkt der Probe bestimmt werden.

Weiters ist eine Steuereinheit 20 vorgesehen, welcher die Messwerte eines die Probentemperatur innerhalb der Messzelle 13 erfassenden Temperatursensors und des Drucksensors zugeführt sind und welche die Kühlleistung der Kühleinrichtung steuert. Die Kühleinrichtung für die Messzelle 13 umfasst beispielhaft eine sich in Kontakt mit der Messzelle 13 befindliche Kühlstufe, die ein Peltier-Element 22 aufweist. Auf der warmen Seite des Peltier-Elements 22 kann eine Kupferplatte 21 angeordnet sein, die mit einem Kühlkörper ausgestattet ist. In der Kupferplatte können Bohrungen bzw. Kanäle ausgebildet sein, durch die eine Kühlflüssigkeit geleitet werden kann.

## Patentansprüche

1. Verfahren zur Bestimmung der Niedertemperatureigenschaften eines paraffinhaltigen Brennstoffs, bei dem der Brennstoff aus einer Vorratskammer (1) durch eine mit einem Sieb (14) versehene Messzelle (13) geleitet wird, die Messzelle (13) mittels einer Kühleinrichtung gekühlt wird, die Temperatur des in der Messzelle (13) befindlichen Brennstoffs gemessen und ein den am Sieb (14) auftretenden Strömungswiderstand repräsentierender Flüssigkeitsdruck gemessen wird und die bei einem definierten Sollwert des Flüssigkeitsdrucks auftretende Temperatur ermittelt und als Ergebnis des Verfahrens ausgegeben wird, **dadurch gekennzeichnet, dass** für die Druckmessung eine definierte Probemenge des Brennstoffs stoßartig aus der Vorratskammer (1) gefördert wird, um einen Druckpuls zu erhalten.

2. Verfahren nach Anspruch 1, **dadurch gekennzeichnet, dass** die Messung des Flüssigkeitsdrucks während des Kühlens der Messzelle (13) bei einer Reihe unterschiedlicher Temperaturen des Brennstoffs wiederholt wird, um eine Messwertreihe zu erhalten, wobei für jede Messung eine definierte, gleiche Probemenge des Brennstoffs stoßartig aus der Vorratskammer (1) gefördert wird, um einen Druckpuls zu erhalten.

3. Verfahren nach Anspruch 1 oder 2, **dadurch gekennzeichnet, dass** das Maximum des bei einem Druckpuls auftretenden Flüssigkeitsdrucks jeweils als Messwert des Flüssigkeitsdrucks herangezogen wird.

4. Verfahren nach Anspruch 1, 2 oder 3, **dadurch gekennzeichnet, dass** der stromaufwärts der Messzelle (13) herrschende Flüssigkeitsdruck als der den am Sieb (14) auftretenden Strömungswiderstand repräsentierende Flüssigkeitsdruck herangezogen wird.

5. Verfahren nach einem der Ansprüche 2 bis 4, **dadurch gekennzeichnet, dass** aus der Messwertreihe eine Kennlinie des Flüssigkeitsdrucks in Abhängigkeit von der Temperatur erstellt wird und die in der Kennlinie dem definierten Sollwert des Flüssigkeitsdrucks zugeordnete Temperatur ermittelt und als Ergebnis des Verfahrens ausgegeben wird.

6. Verfahren nach einem der Ansprüche 1 bis 5, **dadurch gekennzeichnet, dass** die Filtrierbarkeitsgrenze (Cold Filter Plugging Point - CFPP) des Brennstoffs und/oder der Pour Point des Brennstoffs als Ergebnis des Verfahrens ausgegeben wird.

7. Verfahren nach Anspruch 6, **dadurch gekennzeichnet, dass** ein erster Sollwert des Flüssigkeitsdrucks vorgegeben wird, der für die Filtrierbarkeitsgrenze bestimmend ist und dass ein zweiter Sollwert des Flüssigkeitsdrucks vorgegeben wird, der für den Pour Point bestimmend ist.

8. Verfahren nach einem der Ansprüche 1 bis 7, **dadurch gekennzeichnet, dass** die Temperatur des in der Messzelle (13) befindlichen Brennstoffs stufenweise reduziert wird, insbesondere in Stufen von 1°C, und dass nach jeder Abkühlstufe eine Messung des Flüssigkeitsdrucks erfolgt.

9. Verfahren nach einem der Ansprüche 1 bis 7, **dadurch gekennzeichnet, dass** die Temperatur des in der Messzelle (13) befindlichen Brennstoffs kontinuierlich reduziert wird und dass beim Durchlaufen von definierten Temperaturstufen jeweils eine Messung des Flüssigkeitsdrucks erfolgt.

10. Verfahren nach einem der Ansprüche 1 bis 9, **dadurch gekennzeichnet, dass** der Trübungspunkt (Cloud Point) und/oder der Freeze Point des Brennstoffs in der Messzelle (13) mittels eines optischen Messverfahrens ermittelt wird.

11. Verfahren nach Anspruch 10, **dadurch gekennzeichnet, dass** das optische Messverfahren eine Durchlichtmessung umfasst.

12. Verfahren nach einem der Ansprüche 1 bis 11, **dadurch gekennzeichnet, dass** der jeweils in der Messzelle (13) befindliche Brennstoff gekühlt wird und während eines ersten Kühlschrittes der Trübungspunkt und während eines zweiten Kühlschrittes die Filtrierbarkeitsgrenze und/oder der Pour Point ermittelt wird.

13. Verfahren nach Anspruch 12, **dadurch gekennzeichnet, dass** der jeweils in der Messzelle (13) befindliche Brennstoff nach der Kühlung wieder erwärmt wird und während der Erwärmung der Freeze Point ermittelt wird.

14. Vorrichtung zur Durchführung eines Verfahrens nach einem der Ansprüche 1 bis 13, umfassend eine Vorratskammer (1) für den zu prüfenden paraffinhaltigen Brennstoff, eine mit der Vorratskammer (1) in Leitungsverbindung stehende, als Durchflusszelle ausgebildete und mit einem Sieb (14) versehene Messzelle (13), eine Fördervorrichtung zum Fördern von Brennstoff aus der Vorratskammer (1) durch die Messzelle (13), eine Kühlvorrichtung zum Kühlen der Messzelle (13), einen Temperatursensor zum Messen der Temperatur des in der Messzelle (13) befindlichen paraffinhaltigen Brennstoffs, einen Drucksensor zum Messen eines den am Sieb (14) auftretenden Strömungswiderstand repräsentierenden Flüssigkeitsdrucks und eine Steuereinheit mit einer Auswerteschaltung, der die Messwerte des Temperatursensors und des Drucksensors zugeführt sind, wobei die Fördervorrichtung zum stoßartigen Fördern einer definierten Probemenge des paraffinhaltigen Brennstoffs aus der Vorratskammer (1) ausgebildet ist, um einen Druckpuls zu erhalten, und wobei die Auswerteschaltung eingerichtet ist, die bei einem definierten Sollwert des Flüssigkeitsdrucks auftretende Temperatur zu ermitteln und als Ergebnis auszugeben.

15. Vorrichtung nach Anspruch 14, **dadurch gekennzeichnet, dass** die Fördervorrichtung einen die Vorratskammer (1) begrenzenden Kolben (4) umfasst, der mittels einer Antriebseinrichtung betätigbar ist.

16. Vorrichtung nach Anspruch 15, **dadurch gekennzeichnet, dass** die Antriebseinrichtung einen Schrittmotor umfasst.

17. Vorrichtung nach Anspruch 14, **dadurch gekennzeichnet, dass** die Fördervorrichtung eine Mikropumpe oder ein Piezo-Pumpe umfasst.

18. Vorrichtung nach einem der Ansprüche 14 bis 17, **dadurch gekennzeichnet, dass** der Drucksensor zur Messung des stromaufwärts der Messzelle (13) herrschenden Flüssigkeitsdrucks angeordnet ist.

19. Vorrichtung nach Anspruch 18, **dadurch gekennzeichnet, dass** der Drucksensor in den Kolben (4) der Fördervorrichtung integriert ist.

20. Vorrichtung nach einem der Ansprüche 14 bis 19, **dadurch gekennzeichnet, dass** die Steuereinheit mit der Kühlvorrichtung und der Fördervorrichtung zusammenwirkt, um diese in Abhängigkeit von den Messwerten des Temperatursensors und des Drucksensors anzusteuern, und dass die Steuereinheit eingerichtet ist, um die Messung des Flüssigkeitsdrucks während des Kühlens der Messzelle (13) bei einer Reihe unterschiedlicher Temperaturen des Brennstoffs zu wiederholen, wobei eine Messwertreihe erhalten wird, und um für jede Messung eine definierte, gleiche Probemenge des Brennstoffs stoßartig aus der Vorratskammer (1) zu fördern.

21. Vorrichtung nach einem der Ansprüche 14 bis 20, **dadurch gekennzeichnet, dass** das Maximum des bei einem Druckpuls auftretenden Flüssigkeitsdrucks in der Auswerteschaltung jeweils als Messwert des Flüssigkeitsdrucks herangezogen wird.

22. Vorrichtung nach Anspruch 20 oder 21, **dadurch gekennzeichnet, dass** die Auswerteschaltung eingerichtet ist, um aus der Messwertreihe eine Kennlinie des Flüssigkeitsdrucks in Abhängigkeit von der Temperatur zu erstellen und die in der Kennlinie dem definierten Sollwert des Flüssigkeitsdrucks zugeordnete Temperatur zu ermitteln und als Ergebnis auszugeben.

23. Vorrichtung nach einem der Ansprüche 14 bis 22, **dadurch gekennzeichnet, dass** die Steuereinheit mit der Kühlvorrichtung zur stufenweisen Reduzierung der Temperatur des in der Messzelle (13) befindlichen Brennstoffs zusammenwirkt, insbesondere in Stufen von 1°C, und dass nach jeder Abkühlstufe eine Messung des Flüssigkeitsdrucks erfolgt.

24. Vorrichtung nach einem der Ansprüche 14 bis 22, **dadurch gekennzeichnet, dass** die Steuereinheit mit der Kühlvorrichtung zur kontinuierlichen Reduzierung der Temperatur des in der Messzelle (13) befindlichen Brennstoffs zusammenwirkt und dass beim Durchlaufen von definierten Temperaturstufen jeweils eine Messung des Flüssigkeitsdrucks erfolgt.

25. Vorrichtung nach einem der Ansprüche 14 bis 24, **dadurch gekennzeichnet, dass** der Messzelle (13) eine optische Messeinrichtung zur Messung des Trübungspunkts (Cloud Point) und/oder des Freeze Point des Brennstoffs zugeordnet ist.

26. Vorrichtung nach Anspruch 25, **dadurch gekennzeichnet, dass** die optische Messeinrichtung nach dem Durchlichtverfahren arbeitet und vorzugsweise eine an der einen Seite der Messzelle angeordnete Lichtquelle (18) und einen an der gegenüberliegenden Seite der Messzelle angeordneten Lichtsensor (19) umfasst.

## Claims

1. A method for determining the low-temperature properties of a paraffin-containing fuel, in which the fuel is conducted from a storage chamber (1) through a measuring cell (13) provided with a sieve (14), the measuring cell (13) is cooled by means of a cooling device, the temperature of the fuel in the measuring cell (13) is measured, and a fluid pressure representing the flow resistance occurring on the sieve (14) is measured, and the temperature occurring at a defined fluid pressure set point is determined and output as a result of the method, **characterized in that**, for the pressure measurement, a defined sample amount of the fuel is abruptly delivered from the storage chamber (1) in order to obtain a pressure pulse.

2. A method according to claim 1, **characterized in that** the measurement of the fluid pressure during the cooling of the measuring cell (13) is repeated at a number of different temperatures of the fuel in order to obtain a series of measured values, wherein, for each measurement, a defined, identical sample amount of the fuel is abruptly delivered from the storage chamber (1) in order to obtain a pressure pulse.

3. A method according to claim 1 or 2, **characterized in that** the maximum of the fluid pressure occurring at a pressure pulse is each used as the fluid pressure measured value.

4. A method according to claim 1, 2 or 3, **characterized in that** the fluid pressure prevailing upstream of the measuring cell (13) is used as the fluid pressure representing the flow resistance occurring on the sieve (14).

5. A method according to any one of claims 2 to 4, **characterized in that** a characteristic curve of the fluid pressure as a function of the temperature is established from the series of measured values, and the temperature assigned to the defined fluid pressure set point in the characteristic curve is determined and output as a result of the method.

6. A method according to any one of claims 1 to 5, **characterized in that** the cold filter plugging point of the fuel and/or the pour point of the fuel is/are output as a result of the method.

7. A method according to claim 6, **characterized in that** a first fluid pressure set point is specified, which is decisive for the cold filter plugging point, and that a second fluid pressure set point is specified, which is decisive for the pour point.

8. A method according to any one of claims 1 to 7, **characterized in that** the temperature of the fuel in the measuring cell (13) is stepwisely reduced, particularly in steps of 1°C, and that a measurement of the fluid pressure is performed after each cooling step.

9. A method according to any one of claims 1 to 7, **characterized in that** the temperature of the fuel in the measuring cell (13) is continuously reduced, and that a measurement of the fluid pressure is each performed during the passage of defined temperature steps.

10. A method according to any one of claims 1 to 9, **characterized in that** the cloud point and/or the freeze point of the fuel is/are determined in the measuring cell (13) by an optical measuring method.

11. A method according to claim 10, **characterized in that** the optical measuring method comprises a transmitted light measurement.

12. A method according to any one of claims 1 to 11, **characterized in that** the fuel respectively present in the measuring cell (13) is cooled, and the cloud point is determined during a first cooling step and the cold filter plugging point and/or the pour point is/are determined during a second cooling step.

13. A method according to claim 12, **characterized in that** the fuel respectively present in the measuring cell (13) is reheated after cooling, and the freeze point is determined during heating.

14. A device for carrying out a method according to any one of claims 1 to 13, comprising a storage chamber (1) for the paraffin-containing fuel to be tested, a measuring cell (13) in fluid-connection with the storage chamber (1), said measuring cell being designed as a flow cell and provided with a sieve (14), a delivery device for delivering fuel from the storage chamber (1) through the measuring cell (13), a cooling device for cooling the measuring cell (13), a temperature sensor for measuring the temperature of the paraffin-containing fuel in the measuring cell (13), a pressure sensor for measuring a fluid pressure representing the flow resistance occurring on the sieve (14), and a control unit to which the measured values of the temperature sensor and of the pressure sensor are fed, wherein the delivery device is configured to abruptly deliver a defined sample amount of the paraffin-containing fuel from the storage chamber (1) so as to obtain a pressure pulse, and wherein the evaluation circuit is built as to determine, and to output as a result, the temperature occurring at a defined fluid pressure set point.

15. A device according to claim 14, **characterized in that** the delivery device comprises a piston (4) delimiting the storage chamber (1) and operable by a driving device.

16. A device according to claim 15, **characterized in** the driving device comprises a stepper motor.

17. A device according to claim 14, **characterized in that** the delivery device comprises a micropump or a piezo pump.

18. A device according to any one of claims 14 to 17, **characterized in that** the pressure sensor is arranged to measure the fluid pressure prevailing upstream of the measuring cell (13).

19. A device according to claim 18, **characterized in that** the pressure sensor is integrated in the piston (4) of the delivery device.

20. A device according to any one of claims 14 to 19, **characterized in that** the control unit cooperates with the cooling device and with the delivery device to actuate the same as a function of the measured values of the temperature sensor and of the pressure sensor, and that the control unit is arranged to repeat the measurement of the fluid pressure during cooling of the measuring cell (13) at a number of different temperatures of the fuel thus obtaining a series of measured values, and to abruptly deliver, for each measurement, a defined, identical sample amount of the fuel from the storage chamber (1).

21. A device according to any one of claims 14 to 20, **characterized in that** the maximum of the fluid pressure occurring at a pressure pulse is each used as the fluid pressure measured value in the evaluation circuit.

22. A device according to claim 20 or 21, **characterized in that** the evaluation circuit is arranged to establish from the series of measured values a characteristic curve of the fluid pressure as a function of the temperature, and to determine, and output as a result of the method, the temperature assigned to the defined fluid pressure set point in the characteristic curve.

23. A device according to any one of claims 14 to 22, **characterized in that** the control unit cooperates with the cooling device for stepwisely reducing the temperature of the fuel in the measuring cell (13), particularly in steps of 1°C, wherein a measurement of the fluid pressure is performed after each cooling step.

24. A device according to any one of claims 14 to 22, **characterized in that** the control unit cooperates with the cooling device for continuously reducing the temperature of the fuel in the measuring cell (13), and that a measurement of the fluid pressure is each performed during the passage of defined temperature steps.

25. A device according to any one of claims 14 to 24, **characterized in that** an optical measuring device for measuring the cloud point and/or the freeze point of the fuel is associated with the measuring cell (13).

26. A device according to claim 25, **characterized in that** the optical measuring device operates according to the transmitted light method and preferably comprises a light source (18) disposed on one side of the measuring cell and a light sensor (19) disposed on the opposite side of the measuring cell.

## Revendications

1. Procédé pour déterminer les propriétés à basse température d'un combustible paraffinique, au cours duquel le combustible est acheminé d'une chambre de stockage (1) à travers une cellule de mesure (13) dotée d'un tamis (14), la cellule de mesure (13) est refroidie au moyen d'un dispositif de refroidissement, la température du combustible se trouvant dans la cellule de mesure (13) est mesurée et une pression de liquide représentant la résistance à l'écoulement existant au niveau du tamis (14) est mesurée et la température existante à une valeur théorique définie de la pression de liquide est déterminée et est émise en tant que résultat du procédé, **caractérisé en ce que** pour la mesure de pression, une quantité d'échantillon définie du combustible est évacuée par à-coup de la chambre de stockage (1) pour obtenir une impulsion de pression.

2. Procédé selon la revendication 1, **caractérisé en ce que** la mesure de la pression de liquide est répétée pendant le refroidissement de la cellule de mesure (13) lors d'une série de températures différentes du combustible, pour obtenir une série de valeurs mesurées, dans lequel pour chaque mesure, une quantité d'échantillon identique définie du combustible est transportée par à-coup de la chambre de stockage (1) pour obtenir une impulsion de pression.

3. Procédé selon la revendication 1 ou 2, **caractérisé en ce que** la valeur maximale de la pression de liquide existant lors d'une impulsion de pression est utilisée respectivement en tant que valeur mesurée de la pression de liquide.

4. Procédé selon la revendication 1, 2 ou 3, **caractérisé en ce que** la pression de liquide régnant en amont de la cellule de mesure (13) est utilisée comme la pression de liquide représentant la résistance à l'écoulement existant au niveau du tamis (14).

5. Procédé selon l'une quelconque des revendications 2 à 4, **caractérisé en ce qu'**à partir de la série de valeurs mesurées, une courbe caractéristique de la pression de liquide en fonction de la température est produite et la température affectée dans la courbe caractéristique à la valeur théorique définie de la pression de liquide est déterminée et est émise en tant que résultat du procédé.

6. Procédé selon l'une quelconque des revendications 1 à 5, **caractérisé en ce que** la limite de filtrabilité (Cold Filter Plugging Point - CFPP) du combustible et/ou le point d'écoulement du combustible est émis(e) en tant que résultat du procédé.

7. Procédé selon la revendication 6, **caractérisé en ce qu'**une première valeur théorique de la pression de liquide est prédéterminée, qui est déterminant pour la limite de filtrabilité et **en ce qu'**une seconde valeur théorique de la pression de liquide est prédéterminée, qui est déterminante pour le point d'écoulement.

8. Procédé selon l'une quelconque des revendications 1 à 7, **caractérisé en ce que** la température du combustible se trouvant dans la cellule de mesure (13) est réduite par paliers, en particulier par paliers de 1 °C, et **en ce qu'**après chaque palier de refroidissement, une mesure de la pression de liquide est effectuée.

9. Procédé selon l'une quelconque des revendications 1 à 7, **caractérisé en ce que** la température du combustible se trouvant dans la cellule de mesure (13) est réduite en continu et **en ce que** lors du passage de paliers de température définis, une mesure de la pression de liquide est effectuée respectivement.

10. Procédé selon l'une quelconque des revendications 1 à 9, **caractérisé en ce que** le point de trouble (Cloud Point) et/ou le point de congélation du combustible est déterminé dans la cellule de mesure (13) au moyen d'un procédé de mesure optique.

11. Procédé selon la revendication 10, **caractérisé en ce que** le procédé de mesure optique comprend une mesure de transmission de lumière.

12. Procédé selon l'une quelconque des revendications 1 à 11, **caractérisé en ce que** le combustible se trouvant respectivement dans la cellule de mesure (13) est refroidi et pendant une première étape de refroidissement, le point de trouble et pendant une seconde étape de refroidissement, la limite de filtrabilité et/ou le point d'écoulement est déterminé(e).

13. Procédé selon la revendication 12, **caractérisé en ce que** le combustible se trouvant respectivement dans la cellule de mesure (13) est chauffé à nouveau après le refroidissement et pendant le réchauffement, le point de congélation est déterminé.

14. Dispositif pour réaliser un procédé selon l'une quelconque des revendications 1 à 13, comprenant une chambre de stockage (1) pour le combustible paraffinique à tester, une cellule de mesure (13) se trouvant en communication de fluide avec la chambre de stockage (1), conçue comme une cellule de débit et dotée d'un tamis (14), un dispositif de transport pour transporter du combustible de la chambre de stockage (1) à travers la cellule de mesure (13), un dispositif de refroidissement pour refroidir la cellule de mesure (13), un capteur de température pour mesurer la température du combustible paraffinique se trouvant dans la cellule de mesure (13), un capteur de pression pour mesurer une pression de liquide représentant la résistance à l'écoulement existant au niveau du tamis (14) et une unité de commande comportant un circuit d'analyse auquel sont transmises les valeurs mesurées du capteur de température et du capteur de pression, dans lequel le dispositif de transport est conçu pour transporter par à-coup une quantité d'échantillon définie du combustible paraffinique de la chambre de stockage (1) pour obtenir une impulsion de pression, et dans lequel le circuit d'analyse est aménagé pour déterminer la température existant à une valeur théorique définie de la pression de liquide et l'émettre en tant que résultat.

15. Dispositif selon la revendication 14, **caractérisé en ce que** le dispositif de transport comprend un piston (4) limitant la chambre de stockage (1) qui peut être actionné au moyen d'un système d'entraînement.

16. Dispositif selon la revendication 15, **caractérisé en ce que** le système d'entraînement comprend un moteur pas-à-pas.

17. Dispositif selon la revendication 14, **caractérisé en ce que** le dispositif de transport comprend une micro-pompe ou une pompe piézo-électrique.

18. Dispositif selon l'une quelconque des revendications 14 à 17, **caractérisé en ce que** le capteur de pression est agencé pour mesurer la pression de liquide régnant en amont de la cellule de mesure (13).

19. Dispositif selon la revendication 18, **caractérisé en ce que** le capteur de pression est intégré dans le piston (4) du dispositif de transport.

20. Dispositif selon l'une quelconque des revendications 14 à 19, **caractérisé en ce que** l'unité de commande coopère avec le dispositif de refroidissement et le dispositif de transport pour commander ceux-ci en fonction des valeurs mesurées du capteur de température pression et du capteur de pression, et **en ce que** l'unité de commande est aménagée pour répéter la mesure de la pression de liquide pendant le refroidissement de la cellule de mesure (13) lors d'une série de températures différentes du combustible, dans lequel une série de valeurs mesurées est obtenue et pour transporter par à-coup pour chaque mesure, une quantité d'échantillon identique définie du combustible de la chambre de stockage (1).

21. Dispositif selon l'une quelconque des revendications 14 à 20, **caractérisé en ce que** la valeur maximale de la pression de liquide existant lors d'une impulsion de pression est utilisée dans le circuit d'analyse respectivement en tant que valeur mesurée de la pression de liquide.

22. Dispositif selon la revendication 20 ou 21, **caractérisé en ce que** le circuit d'analyse est aménagé pour produire à partir de la série de valeurs mesurées, une courbe caractéristique de la pression de liquide en fonction de la température et pour déterminer la température affectée dans la courbe caractéristique à la valeur théorique définie de la pression de liquide et l'émettre en tant que résultat.

23. Dispositif selon l'une quelconque des revendications 14 à 22, **caractérisé en ce que** l'unité de commande coopère avec le dispositif de refroidissement pour réduire par paliers la température du combustible se trouvant dans la cellule de mesure (13), en particulier par paliers de 1 °C, et **en ce qu'**après chaque palier de refroidissement, une mesure de la pression de liquide est effectuée.

24. Dispositif selon l'une quelconque des revendications 14 à 22, **caractérisé en ce que** l'unité de commande coopère avec le dispositif de refroidissement pour réduire en continu la température du combustible se trouvant dans la cellule de mesure (13), et **en ce que** lors du passage de paliers de température définis, une mesure de la pression de liquide est effectuée respectivement.

25. Dispositif selon l'une quelconque des revendications 14 à 24, **caractérisé en ce qu'**à la cellule de mesure (13) est affecté un système de mesure optique pour la mesure du point de trouble (Cloud point) et/ou du point de congélation du combustible.

26. Dispositif selon la revendication 25, **caractérisé en ce que** le système de mesure optique travaille selon le procédé de transmission de lumière et comprend de préférence une source de lumière (18) disposée au niveau d'un côté de la cellule de mesure et un capteur de lumière (19) disposé au niveau du côté opposé de la cellule de mesure.
